(19) Europäisches Patentamt
European Patent Office
Office européen des brevets

(11) Veröffentlichungsnummer: **0 229 897**
**A1**

(12) # EUROPÄISCHE PATENTANMELDUNG

(21) Anmeldenummer: **86114987.0**

(22) Anmeldetag: **28.10.86**

(51) Int. Cl.⁴: **C 07 D 473/08**, A 61 K 31/52

(30) Priorität: **26.12.85 US 813439**

(43) Veröffentlichungstag der Anmeldung: **29.07.87 Patentblatt 87/31**

(84) Benannte Vertragsstaaten: **AT BE CH DE ES FR GB GR IT LI LU NL SE**

(71) Anmelder: **SIEGFRIED AKTIENGESELLSCHAFT, CH-4800 Zofingen (CH)**

(72) Erfinder: **Thiele, Kurt, Dr., Rebbergstrasse 47d, CH-4800 Zofingen (CH)**
Erfinder: **Geissmann, Felix, Brittnauerstrasse 28, CH-4800 Zofingen (CH)**
Erfinder: **Zirngibl, Ludwig, Dr., Eisgrubenweg 16, CH-4800 Zofingen (CH)**
Erfinder: **Jahn, Ulrich, Dr., Kirchmoostrasse 13, CH-4800 Zofingen (CH)**

(74) Vertreter: **Jaeger, Klaus, Dr. et al, Jaeger & Partner Patentanwälte Pippinplatz 4a, D-8035 München-Gauting (DE)**

(54) **Alpha1-adrenerg blockierende Theophyllinderivate.**

(57) Neue α1-adrenerg blockierende Theophyllinderivate, zum Beispiel
7-(4-(4-(p-Fluorbenzoyl)piperidino)-
butyltheophyllin,
werden als blutdrucksenkende Arzneimittel beschrieben. Diese Stoffe können ausgehend von Theophyllin durch Alkylierung mit einem Dihalogenalkan und anschließender Umsetzung mit 4-(p-Fluorbenzoyl)piperidin synthetisiert werden.

EP 0 229 897 A1

JAEGER & PARTNER
PATENTANWÄLTE

27. Oktober 1986

Siegfried Aktiengesellschaft
CH-4800 Zofingen

SGF-107-EP
Ba/zi

# α1-adrenerg blockierende Theophyllinderivate

## Beschreibung

Die Erfindung betrifft neue Theophyllinderivate mit α1-adrenerg blockierender Wirkung sowie ein Verfahren zur Herstellung solcher Stoffe.

Pharmokologisch interessante Theophyllinderivate sind schon seit langem bekannt. Beispielsweise wurden Präparate mit dem Theophyllingrundgerüst zur Behandlung von Asthma- oder Kreislauferkrankungen eingesetzt. Die Patentschrift EP 71 738 B1 offenbart in 7-Stellung durch Alkylreste substituierte Theophyllinderivate, wobei die Alkylreste ihrerseits derivatisierte Piperidinsubstituenten aufweisen. Als Beispiel sei in diesem Zusammenhang 7-(2-(4-(p-Fluorbenzoyl)-piperidino)ethyl)theophyllin genannt. Die erwähnten Theo-

BERGSTRASSE 48½ · D-8035 MÜNCHEN-GAUTING
TELEPHON: (089) 8502030 · TELEX: 521777 isar d

phyllinderivate weisen ein relativ breites und unspezifisches Wirkungsspektrum auf. Die bekannten Stoffe können histamin-, serotonin- und bradykinin-antagonistische, blutdrucksenkende, antianaphylaktische und ß-adrenergstimulierende Eigenschaften zeigen. Sie werden daher als Migränemittel, Broncholytika, Antiallergika, Antiphlogistika, Analgetika und Antihypertonika verwendet.

Das breite Wirkungsspektrum dieser Stoffe ist insofern vorteilhaft, als diese vielseitig einsetzbar sind, andererseits aber auch nachteilig, da die Wirkung wenig spezifisch ist und sich somit ungewollte Nebenwirkungen einstellen können. In diesem Zusammenhang ist von Interesse, daß typische Vertreter der oben genannten Verbindungen eine ausgeprägte Hemmwirkung bezüglich des Neurotransmitters Serotonin entfalten.

Angesichts dieses Standes der Technik liegt der Erfindung die Aufgabe zugrunde, neue Stoffe bzw. Arzneimittel zu schaffen, die eine selektiv α1-adrenerg blockierende Wirkung zeigen.

Es wird also vorgeschlagen, Theophyllinderivate der Formel

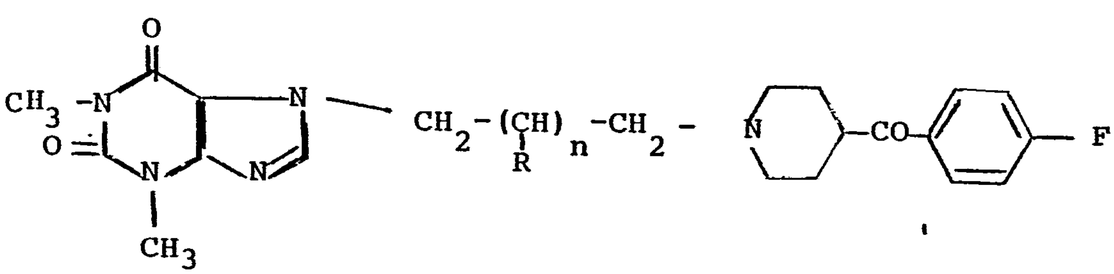 (I)

in der R Wasserstoff oder eine Hydroxygruppe und n 2 oder 3 ist, zu schaffen. Die Stoffe können als solche oder in Form pharmazeutisch zulässiger Säureadditionssalze angewandt werden.

Die vorgeschlagenen Substanzen zeichnen sich durch eine ausgeprägte α1-adrenerg blockierende Wirkung aus, die etwa der bereits bekannter α1-adrenerg blockierender Wirkstoffe wie Prazosin entspricht. Gleichzeitig ist die antagonistische Wirkung gegen Serotonin relativ klein, so daß die α1-adrenerg blockierende Wirkung selektiv ist. Dieses Ergebnis ist im Hinblick auf die ß-stimulierende Wirkung und den deutlichen serotoninantagonistischen Effekt der Verbindungen Sgd 144-80 und Sgd 195-78 (Codebezeichnung aus EP 71 738 B1) gerade wegen der strukturellen Ähnlichkeit überraschend.

Die genannten pharmakologischen Wirkungen der erfindungsgemäßen Verbindung prädestinieren diese als selektiv wirksame blutdrucksenkende Mittel.

Die Stoffe der Erfindung können ausgehend von Theophyllin durch Umsetzung mit einem 1,4-Dibutyl- bzw. 1,5-Dipentylhalogenid oder einem entsprechenden Tosylat und anschließender Aminierung mit 4-(p-Fluorbenzoyl)piperidin erhalten werden. Diese Reaktionssequenz ist bereits aus Arzneim.-Forsch. 27,5ff. (1977) bekannt.

Durch die Verwendung von Butyl- bzw. Pentylderivaten mit unterschiedlichen Abgangsgruppen kann die Selektivität des ersten Reaktionsschrittes, der Alkylierung von Theophyllin, begünstigt werden, da in diesem Fall nur sehr wenig Ditheophyllinbutan bzw. Ditheophyllinpentan erhalten wird.

Zu den bevorzugten difunktionalisierten Butan- bzw. Pentanderivaten gehören 1,4-Dibrombutan, 1,4-Bromchlorbutan, 1,5-Dibrompentan und 1,5-Bromchlorpentan.

Ein geeignetes Lösungsmittel für den ersten Reaktionsschritt ist Dimethylformamid, dem gegebenenfalls eine anorganische

Base wie Kaliumcarbonat zugesetzt werden kann. Bei Verwendung von genügend reaktiven Butan- bzw. Pentanderivaten kann bei Temperaturen im Bereich zwischen 30 und 40 °C gearbeitet werden.

Das erhaltene Zwischenprodukt

$$Th-CH_2-(\underset{\displaystyle R}{\underset{|}{CH}})_n-CH_2-Hal$$

(Th steht für den Theophyllinrest) wird dann mit 4-(p-Fluorbenzoyl)piperidin umgesetzt. Die Reaktion kann bei erhöhter Temperatur mit oder ohne Verwendung eines Lösungsmittels (wie z.B. Wasser) herbeigeführt werden. Beim Arbeiten ohne Lösungsmittel werden die Ausgangsstoffe solange auf der Temperatur der Mischschmelz gehalten bis periodisch entnommene Proben des Reaktionsgemisches bei Kontrolle mittels Dünnschicht-Chromatographie erkennen lassen, daß die Reaktion im wesentlichen abgeschlossen ist. Mit Vorteil benützt man dabei zur Bindung des abgespaltenen Halogenwasserstoffs einen Protonenakzeptor, z.B. Kaliumcarbonat oder das als Ausgangsstoff verwendete Piperinderivat.

## Beispiel 1

7-(4-(4-(p-Fluorbenzoyl)piperidino)butyltheophyllin (Sgd1-85)

45 g Theophyllin, 145 ml N,N-Dimethylformamid, 34,6 g Kaliumcarbonat und 59,4 g Dibrombutan werden in einem 1 l-Glaskolben auf 35 °C erhitzt. Die so erhaltene Suspension wird 6 h bei 35 °C gerührt. Zum Reaktionsgemisch werden anschließend 270 ml Wasser zugesetzt und das Gemisch bei 4 °C in einem Kühlschrank über Nacht gelagert. Die so erhaltenen Kristalle werden von der flüssigen Phase durch Vakuumfiltration abgetrennt,

mit 60 ml Wasser gewaschen und auf dem Filter bei anhaltendem filtratseitigen Vakuum getrocknet.

Da in diesem Ausführungsbeispiel anstelle des teuereren, aber selektiveren 1,4 Bromchlorbutan das Dibromderivat verwendet wurde, wird auch etwas Ditheophyllinbutan neben dem gewünschten 7-(4-Brombutyl)theophyllin erhalten. Zur Reinigung des hohen 7-(4-Brombutyl)theophyllins werden die Kristalle in 400 ml Ethanol aufgenommen und auf 50 °C unter Rühren erhitzt. Das Rühren wird 20 min bei 50 °C fortgesetzt. Die noch warme Suspension wird abfiltriert und mit 50 ml Ethanol gewaschen. Die so erhaltene abfiltrierte ethanolische Lösung wird anschließend zur Trocknung eingeengt und der zurückbleibende Feststoff mit 150 ml n-Hexan vermischt. Nach Waschen der Suspension wird die flüssige Phase nochmals abfiltriert, während die zurückbleibenden Kristalle mit 50 ml n-Hexan gewaschen werden.

Das gereinigte Produkt hat einen Schmelzpunkt von 96 °C. Es wird in Form eines weißen pulverförmigen Stoffes in einer Menge von 27,4 g erhalten.

Das 7-(4-Brombutyl)theophyllin wird dann mit 4-(Fluorbenzoyl)piperidin zur Reaktion gebracht. Zu diesem Zweck werden 28,5 g 4-(Fluorbenzoyl)piperidin in 900 ml heißem Wasser von etwa 55 °C in einem 1 l-Glaskolben gelöst. Unter Rühren werden 0,7 g Aktivkohle zugesetzt. Nach Abfiltrieren der Aktivkohle erhält man eine hellgelbe Lösung. Diese Lösung wird in einen 2 l Glaskolben überführt und unter Rühren mit 20,1 g Kaliumcarbonat und 30 g 7-(4-Brombutyl)theophyllin versetzt. Dann wird das Reaktionsgemisch auf 100 °C erhitzt und bei dieser Temperatur 3 h gehalten. Nach Abkühlung auf etwa 50 °C werden 250 ml iso-Butylmethylketon in das Reaktionsgemisch gegeben. Nach Phasentrennung wird die untere wässrige Phase von der

oberen organischen Phase abgetrennt. Die organische Phase wird zum Trocknen unter vermindertem Druck eingeengt. Der so erhaltene Feststoff wird in Petrolether aufgenommen und der Feststoff aus der Suspension durch Vakuumfiltration abgetrennt. Man erhält 21 g Rohprodukt.

Das Rohprodukt wird durch Säulenchromatographie auf Kieselgel mit Chloroform/Methanol (9:1) als Laufmittel gereinigt und schließlich zweimal aus Methanol umkristallisiert. Man erhält 13 g der Titelverbindung in hochreiner Form mit einem Schmelzpunkt von 137 °C.

Beispiel 2

7-(5-(4-(p-Fluorbenzoyl)-piperidino)-pentyl)-theophyllin (Sgd 2-85)

Das Verfahren aus Beispiel 1 wird wiederholt mit Ausnahme, daß 63,3 g 1,5-Dibrompentan anstelle von 59,4 g 1,4-Dibrombutan mit Theophyllin umgesetzt werden.

Nach chromatographischer Reinigung und zweifacher Umkristallisation des Rohproduktes aus Methanol werden 15,8 g der Titelverbindung in einer Reinheit von 99 % erhalten. Das Produkt hat einen Schmelzpunkt von 125 °C.

JAEGER & PARTNER
PATENTANWÄLTE

27. Oktober 1986

Siegfried Aktiengesellschaft
CH-4800 Zofingen

SGF-107-EP
Ba/zi

α1-adrenerg blockierende Theophyllinderivate

P a t e n t a n s p r ü c h e

für die Vertragsstaaten BE, DE, FR, IT, LI, LU, NL, SE, CH, GB.

1. Theophyllinderivate der Formel (I)

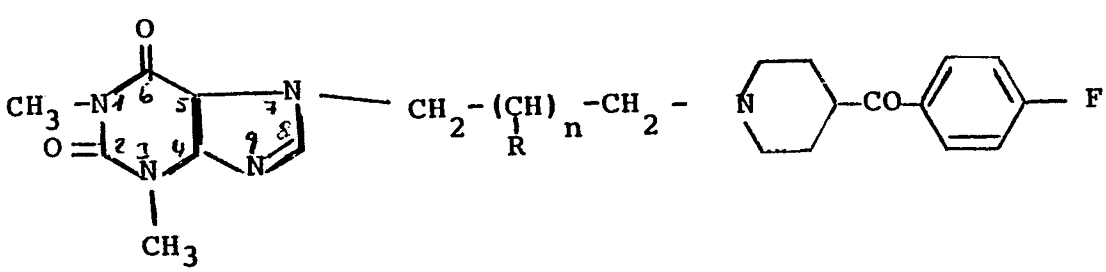
(I)

BERGSTRASSE 48½ · D-8035 MÜNCHEN-GAUTING
TELEPHON: (089) 8502030 · TELEX: 521777 isar d

und ihre Salze mit pharmazeutisch zulässigen Säuren, wobei R Wasserstoff oder eine Hydroxygruppe und n 2 oder 3 ist.

2. Theophyllinderivate nach Anspruch 1,
dadurch g e k e n n z e i c h n e t,
daß das Theophyllinderivat 7-(4-(4-Fluorbenzoyl)-piperidino)-butyl)theophyllin ist.

3. Theophyllinderivat nach Anspruch 1,
dadurch g e k e n n z e i c h n e t,
daß das Theophyllinderivat 7-(5-(4-Fluorbenzoyl)-piperidino)pentyl)theophyllin ist.

4. Arzneimittel mit α1-adreneger Wirkung,
dadurch g e k e n n z e i c h n e t,
daß es ein Theophyllinderivat nach Anspruch 1 enthält.

5. Arzneimittel nach Anspruch 4,
dadurch g e k e n n z e i c h n e t,
daß es 7-(4-(4-Fluorbenzoyl)-piperidino)butyl)theophyllin enthält.

6. Arzneimittel nach Anspruch 4,
dadurch g e k e n n z e i c h n e t,
daß es 7-(5-(4-Fluorbenzoyl)-piperidino)pentyl)theophyllin enthält.

7. Verwendung eines Theophyllinderivates nach Anspruch 1 zur Herstellung eines Arzneimittels mit α1-adrenerger Wirkung.

8. Verwendung eines Theophyllinderivates nach Anspruch 7,
dadurch g e k e n n z e i c h n et,

daß das Theophyllinderivat 7-(4-(4-Fluorbenzoyl)-piperidino)butyl)theophyllin ist.

9. Verwendung eines Theophyllinderivates nach Anspruch 7,
dadurch gekennzeichnet,
daß das Theophyllinderivat 7-(5-(4-Fluorbenzoyl)-piperidino)pentyl)theophyllin ist.

10. Verfahren zur Herstellung der Theophyllinderivate von Anspruch 1,
dadurch gekennzeichnet,
daß man in 7-Stellung mit einer Gruppe der Formel

$$-CH_2-(\underset{\displaystyle R}{\overset{|}{C}}H)_n-CH_2-Hal$$

substituiertes Theophyllin (wobei R und n die oben genannte Bedeutung haben und "Hal" Chlor oder Brom bedeuten) mit einer Base der Formel

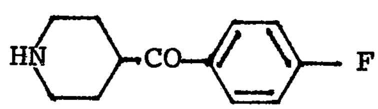

zur Reaktion bringt.

JAEGER & PARTNER
PATENTANWÄLTE

0229897

27. Oktober 1986

Siegfried Aktiengesellschaft
CH-4800 Zofingen

SGF-107-EP
Ba/zi

α1-adrenerg blockierende Theophyllinderivate

P a t e n t a n s p r ü c h e

für die Vertragsstaaten AT, ES und GR

1. Verfahren zur Herstellung von Theophyllinderivaten, dadurch g e k e n n z e i c h n e t, daß man zur Herstellung von Derivaten der Formel

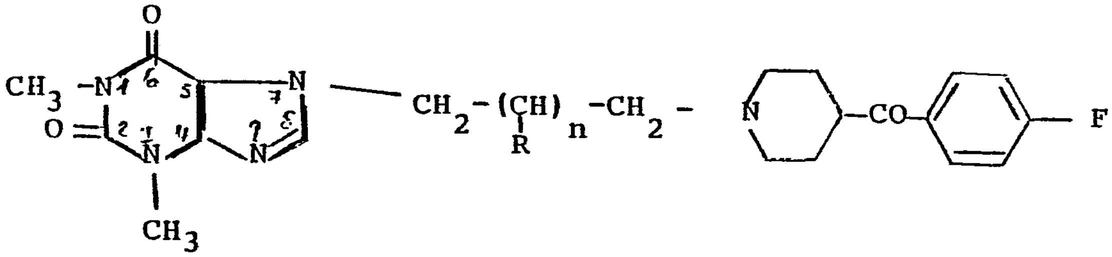

(I)

BERGSTRASSE 48½ · D-8035 MÜNCHEN-GAUTING
TELEPHON: (089) 8502030 · TELEX: 521777 Isar d

und ihre Salze mit pharmazeutisch zulässigen Säuren, wobei R Wasserstoff oder die Hydroxylgruppe und n 2 oder 3 bedeutet, in 7-Stellung mit einer Gruppe der Formel

$$-CH_2-\underset{R}{(CH)}_n-CH_2-Hal$$

substituiertes Theophyllin (wobei R und n die oben genannte Bedeutung haben und "Hal" Chlor oder Brom bedeutet) mit einer Base der Formel

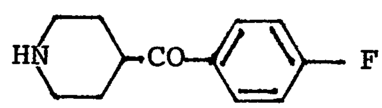

zur Reaktion bringt.

2. Verfahren nach Anspruch 1,
dadurch gekennzeichnet,
daß das Theophyllinderivat (I) 7-(4-(4-Fluorbenzoyl)-piperidino)butyl)theophyllin ist.

3. Verfahren nach Anspruch 1,
dadurch gekennzeichnet,
daß das Theophyllinderivat 7-(5-(4-Fluorbenzoyl)-piperidino)pentyl)theophyllin ist.

0229897

Europäisches Patentamt

# EUROPÄISCHER RECHERCHENBERICHT

Nummer der Anmeldung

EP 86114987.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP - A1 - 0 071 738 (SIEGRIED AKTIENGESELLSCHAFT)<br>* Seite 3; Beispiele 2-4 *<br>-- | 1,4,10 | C 07 D 473/08<br>A 61 K 31/52 |
| A | FR - M - 5 086 (CHUGAI SEIYAKU KABUSHIKI KAISHA)<br>* Seite 1; Beispiel 46 *<br>---- | 1,4,10 | |

| RECHERCHIERTE SACHGEBIETE (Int. Cl.4) |
|---|
| C 07 D 473/00 |

Der vorliegende Recherchenbericht wurde für alle Patentansprüche erstellt.

| Recherchenort | Abschlußdatum der Recherche | Prüfer |
|---|---|---|
| WIEN | 02-02-1987 | HERING |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82